(19) Europäisches Patentamt
European Patent Office
Office européen des brevets

(11) Publication number: 0 174 371
B1

(12) EUROPEAN PATENT SPECIFICATION

(45) Date of publication of patent specification: 19.09.90

(51) Int. Cl.5: C 09 B 11/12, C 12 Q 1/28, G 01 N 33/50

(21) Application number: 84902363.5

(22) Date of filing: 12.06.84

(86) International application number: PCT/JP84/00305

(87) International publication number: WO 85/03942 12.09.85 Gazette 85/20

(60) Divisional application 89117475.7 filed on 12/06/84.

# (54) TRIPHENYLMETHANE DERIVATIVES AND METHOD FOR DETERMINING OXIDATIVE SUBSTANCES USING THEM AS COLOR-FORMING COMPONENT.

(30) Priority: 02.03.84 JP 40031/84
15.03.84 JP 49950/84
02.04.84 JP 65629/84
10.04.84 JP 71548/84

(43) Date of publication of application: 19.03.86 Bulletin 86/12

(45) Publication of the grant of the patent: 19.09.90 Bulletin 90/38

(84) Designated Contracting States: AT BE CH DE FR GB LI LU NL SE

(56) References cited:
FR-A-2 099 783
JP-A-5 374 530
JP-A-5 429 335
JP-A-5 582 158
JP-B-5 212 189
US-A-4 008 267

(73) Proprietor: WAKO PURE CHEMICAL INDUSTRIES, LTD.
10, Doshomachi-3-chome
Higashi-ku Osaka (JP)

(72) Inventor: YAMANISHI, Kazuhiko 17-10, Akatuka 3-chome
Itabashi-ku
Tokyo 175 (JP)
Inventor: SHINTANI, Akinori 6-43-4, Higashisonodacho
Amagasaki-shi
Hyougo 661 (JP)
Inventor: OKAJIMA, Satoru 6-15-6, Higashisonodacho
Amagasaki-shi
Hyougo 661 (JP)
Inventor: HANADA, Toshiro 103, Minami Haitu 784, Oaza Minamiotuka
Kawagoe-shi Saitama 350 (JP)

(74) Representative: Dr. Elisabeth Jung Dr. Jürgen Schirdewahn Dipl.-Ing. Claus Gernhardt
P.O. Box 40 14 68 Clemensstrasse 30
D-8000 München 40 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 174 371 B1

Courier Press, Leamington Spa, England.

## Description

Technical Field

The present invention relates to a triphenyl methane derivative, and a method of quantitatively measuring an oxidative substance by using said derivative as a coloring component.

Background Technique

Since changes in components of body fluids such as blood, urine and the like are closely correlated with diseases, the measurement of the body fluid components has become important in order to diagnose the diseases, to solve pathological conditions and to select a therapeutic course. For instance, it is well known that there have been developed methods of measuring a great variety of micro-components including cholesterol, triglyceride, glucose, uric acid, monoamine oxidase, bile acid and the like in the blood, and they have been used for the diagnosis of the diseases.

At present, as a method of measuring serum components there is generally popularized a so-called "enzyme method" in which an enzyme reaction is carried out by using an enzyme specifically acting upon a target component, and a product formed thereby is measured to determine the content of the target component. With the development of oxidizable coloring reagents, there has been developed and used, for example, a method in which an oxidizable coloring reagent is oxidized with a peroxidase-like substance and an oxidative substance to form a coloring system. Representative for this technique is a method in which an $H_2O_2$ generating enzyme, for instance, an oxidase, is used to generate $H_2O_2$ in an amount necessary for the respective target component and the thus generated $H_2O_2$ is forming a coloring system with a peroxidase and an oxidizable coloring reagent as a coloring component, the amount of the target component being determined through colorimetric determination. By way of example there may be recited a method in which $H_2O_2$ is generated by the combined use of cholesterol-cholesterol oxidase, glucose-glucose oxidase, triglyceride-lipoprotein lipase-glycerol oxidase, uric acid-uricase, or the like, forming a coloring system by using peroxidase and an oxidizable coloring reagent, the color development being measured by using a photometer to determine the amount of a target component. As a matter of course, the concentrations of the components in the serum differ depending upon the kinds of the components, and therefore the amount of $H_2O_2$ generated through the enzyme reaction varies over a wide range. Accordingly, there have been developed and used oxidizable coloring reagents with sensitivities meeting the intended purposes. For example, oxidizable coloring reagents combining 4-aminoantipyrine with a phenolic compound of a N,N-dialkylaniline compound are generally used in the measurement of cholesterol, triglyceride, glucose, uric acid or the like.

However, among the body fluid components, there are some components which are contained in the normal serum in an extremely small amount, like monoamine oxidase and bile acid. The monoamine oxidase is an enzyme which acts upon a monoamine compound to generate $H_2O_2$ and aldehyde, but the amount of the generated $H_2O_2$ is extremely small due to its extremely small concentration in the serum. Thus, the oxidizable coloring reagents of the above combinations are insufficient in sensitivity to quantitatively determine such components. Consequently, an oxidizable coloring reagent with a higher sensitivity is being demanded. Under these circumstances, there has heretofore been developed and used on a commercial basis a measuring method using a derivative of leucomethylene blue as the oxidizable coloring reagent with a higher sensitivity. However, this leuco coloring matter has the defect that its stability in a solution is insufficient, and therefore a coloring reagent containing this leuco coloring matter is gradually colored during storage.

Furthermore, although for similar purposes there have been studied a leucocrystal violet, a leuco malachite green and so on as an oxidizable coloring reagent with high sensitivity which belong to the same triphenyl methane type leuco coloring matters as in the present invention, all of them are difficult to dissolve into water in the neutral pH-range. Therefore, it is difficult to dissolve them in a desired concentration. Thus, they are unsuitable for the measurement of a micro-component.

As a leuco coloring matter improved in its water solubility, there has been proposed bis(4-diethylaminophenyl)-2-sulfophenyl methane (hereinafter abbreviated as BSPM) (Japanese Patent Application Laid-Open No. 26199/1981). However, it can not necessarily be said that the solubility thereof in water is sufficient.

Therefore, it is an object of the present invention to provide a novel coloring reagent which is excellent as regards solubility in water in the neutral pH-range, stable as an aqueous solution for a long period of time, has a high coloring sensitivity and is stable in coloring, exhibits a coloring having an absorption at a longer wavelength, and is accordingly less susceptible to the influence of a component in a serum.

It is another object of the present invention to provide a method of quantitatively measuring an oxidative substance by using this coloring reagent.

Disclosure of the Invention

The present invention relates to a novel triphenyl methane derivative represented by the following general formula (I):

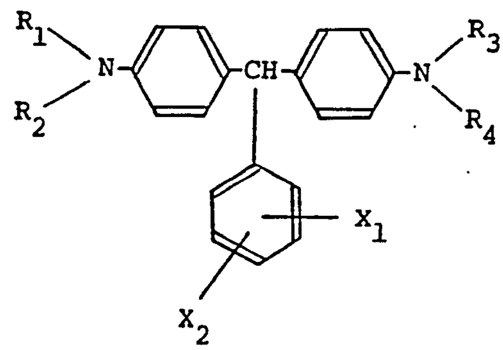

(I)

wherein $R_1$, $R_2$, $R_3$ and $R_4$, which may be the same as or different from one another, represent $C_{1-3}$-alkyl groups, and $X_1$ and $X_2$ both represent $-O(CH_2)_nSO_3M$ in which M is a hydrogen atom, an alkali metal ion or $NH_4^+$, and n is an integer of 2—4, or either of $X_1$ and $X_2$ represents $-O(CH_2)_nSO_3M$ in which M and n are the same meanings as given above and the other represents a hydrogen atom, and a method of quantitatively measuring the amount of an oxidative substance by using said triphenyl methane derivatives as a coloring component.

In the triphenyl methane derivatives represented by the general formula (I) according to the present invention, $R_1$—$R_4$ represent alkyl groups such as methyl, ethyl, propyl, etc., and they may be the same as or different from one another. M in $-O(CH_2)_nSO_3M$ is a hydrogen atom, an alkali metal ion such as $Na^+$, $K^+$, $Li^+$, etc., or $NH_4^+$, and n is 2, 3 or 4.

It is considered that the triphenyl methane derivatives represented by the general formula (I) according to the present invention are novel compounds which have not been described in the literature, and the triphenyl methane derivatives may generally be produced by the following process.

For instance, in the case of bis(4-N,N-diethylaminophenyl)-3,4-disodium sulfopropoxyphenyl methane, protocatechualdehyde and propane sultone are reacted with each other under heating in an organic solvent such as methyl cellosolve, ethyl cellosolve or the like in the presence of an alkali such as caustic soda, sodium alcoholate or the like, and after the completion of the reaction, ordinary post-treatments such as cooling, crystal precipitation, solvent pouring, filtration, washing and so on are carried out, and if necessary, purification by a column chromatography or the like is carried out to obtain 3,4-disodium sulfopropoxybenzaldehyde. Then, this aldehyde is reacted with N,N-diethylaniline in an organic solvent such as methyl cellosolve, ethyl cellosolve or the like under heating in the presence of a catalyst such as zinc chloride or the like, and post-treatments are carried out in the ordinary way and purification is carried out by a column chromatography or the like to obtain the intended product. Generally, the other compounds encompassed by the general formula (I) may be produced in the same or similar manner.

The triphenyl methane type leuco coloring matters represented by the general formula (I) according to the present invention have first been synthesized by the present inventors to improve the inferior solubility to water in a neutral pH-range, which is the defect of the known triphenyl methane type leuco coloring matters such as leucomalachite green, leucocrystal violet and so on. It has the characteristic feature that a group $-O(CH_2)_nSO_3M$ (M and n represent the same meanings as given above) is introduced as a solubilizing group.

The above-mentioned BSPM is a triphenyl methane derivative in which one sulfonic acid group is introduced into a benzene nucleus to improve the solubility of the triphenyl methane type leuco coloring matter, but its solubility at pH 7.2 is only 0.5 mM. Thus, it cannot be said that the solubility in water around the neutral pH-range is sufficient. As regards compound bis(4-diethylaminopheny)-2,4-disulfophenyl methane in which two sulfonic acid groups are introduced into a benzene nucleus, although the solubility in water is obviously improved, it cannot be oxidized in a $H_2O_2$—POD (peroxidase) system due to its high oxidation-reduction potential, so that no coloring takes place. Therefore, this compound cannot be used in a body fluid component-measuring method in which $H_2O_2$ is generated by using an enzyme, forming a coloring system for the measurement of a target component.

In contrast thereto the triphenyl methane derivative of the present invention, even those comprising only a single group $-O(CH_2)_nSO_3M$ (M and n are the same as given above), for example, bis(4-N,N-diethylaminophenyl)-4-sodium sulfopropoxyphenyl methane (hereinafter abbreviated as BSproPM) exhibits a solubility in water of 5mM around the neutral range (pH 7.2) which is 10 times as large as that of BSPM. The derivatives having two $-O(CH_2)_nSO_3M$ groups, for example, bis(4-N,N-diethylaminophenyl)-3,4-disodium sulfopropoxyphenyl methane (hereinafter abbreviated as BSdiproPM) is even more soluble and exhibits a solubility of not less than 30 times (15 mM or more) as that of BSPM.

The triphenyl methane derivatives according to the present invention in which two groups $-O(CH_2)_nSO_3M$ (M and n are the same meanings as given above), are present, are unexpectedly different

from the triphenyl methane derivative in which two sulfonic acid groups are directly bonded to the benzene nucleus, since they are excellent in oxidation coloring in the $H_2O_2$—POD system, and can be satisfactorily used for the purpose aimed at by the present invention.

The leuco coloring matter according to the present invention is extremely stable in form of a solution state. While an aqueous solution of the conventional leucomethylene blue derivative becomes unusable at room temperature in several hours, the aqueous solution of the leuco coloring matter according to the present invention does not change at all even during a 24 hour storage, and therefore it is exceedingly useful as coloring component.

Every leuco coloring reagent according to the invention has a coloring sensitivity of as high as 80,000, and uniformly has the $\lambda_{max}$ value on a long wave length side, that is, at 600—700 nm, so that they are hardly susceptible of interference with hemoglobin, bilirubin and the like.

Further, coloring caused by the coloring matters according to the invention is extremely stable, and almost no color fading is observed.

As mentioned above, since the triphenyl methane derivatives according to the invention have various merits, the derivatives can be effectively used in the known measuring method in which the triphenyl methane derivative is oxidized with an oxidative substance such as $H_2O_2$ and peroxidase or a peroxidase-like substance such as hemoglobin to form a coloring system thereby quantitatively determining the amount of the target component, for example, in the case of the measurement of the body fluid components such as blood, urine and the like according to the enzyme method ($H_2O_2$ generating system), and in the measurement of hemoglobin in the serum by using $H_2O_2$ or an oxidative substance such as sodium perborate. As the chemical components in body fluids which can be measured by the method of the invention, mention may be made of all target compounds which are measured according to the conventional enzyme method ($H_2O_2$ generating system), for instance, glucose, free cholesterol, whole cholesterol, HDL (high specific density lipoprotein)-cholesterol, LDL (low specific density lipoprotein)-cholesterol, triglyceride, phospholipid, uric acid, monoamine oxidase and so on (in the case of uric acid, it is necessary that uricase is preliminarily acted upon uric acid to generate $H_2O_2$, and then the coloring reagent according to the present invention is added together with peroxidase for color development). The coloring reagent according to the present invention is particularly suited for the measurement of a component contained in the body fluids in an extremely small amount, for example, monoamine oxidase, and bile acid.

In practicing the method of the invention with respect to the serum, a concentration of more than 0.01 mM of the triphenyl methane type oxidizable coloring reagent of the invention is suitable, and in general a concentration of 0.02—0.3 mM is preferably used.

Brief Description of the Drawings

Fig. 1 shows a calibration curve obtained in Example 3 in which absorbances (OD) are plotted on an ordinate and the respective monoamine oxidase activities (IU/l) on an abscissa, and plotted points are connected;

Fig. 2 shows a calibration curve obtained in Example 4 and Reference Example 2 respectively (—●—●— being the calibration curve in Example 4 and x—x being the one in Reference Example 2) in which absorbances (OD) are plotted on an ordinate and the respective cholesterol concentrations (mg/dl) on an abscissa and the plotted points are connected;

Fig. 3 shows a calibration curve obtained in Example 5 in which absorbances (OD) are plotted on an ordinate and the respective hydrogen peroxide concentrations (ppm) on an abscissa, and the plotted points are connected;

Fig. 4 shows a calibration curve obtained in Example 6 in which absorbances (OD) are plotted on an ordinate and the respective hydrogen peroxide concentrations (ppm) on an abscissa, and the plotted points are connected.

Best Mode for Practicing the Invention

Example 1

Synthesis of BSdiproPM

(i) Synthesis of 3,4-disodium sulfopropoxybenzaldehyde

To 27.6 g (0.2 mole) of protocatechualdehyde dissolved in 200 ml of methanol was added 92.4 g (0.48 mol) of 28% sodium methylate, which mixture was concentrated to dryness. 400 ml of methyl cellosolve was added to the thus dried matter, which was dissolved under stirring. Then, 58.8 g (0.48 mol) of propanesultone dissolved in 50 ml of methyl cellosolve were dropwise added to the solution at 95—100°C, and after the completion of addition, the reaction was carried out at the same temperature under stirring for one hour. After cooling of the reaction liquid, acetone was added thereto to precipitate crystals, and the crystals were filtered out, followed by drying, to obtain 88 g of crude crystals (yield 103.2%). The crude product was purified by a colum chromatography (ODS reversed phase column chromatography, 20% methanol aqueous solution containing 5% of AcOH) to obtain 43.5 g of purified crystals. TLC: one spot, IR (KBr): $\nu$ = 1055 (—$SO_3^{\ominus}$, $\phi$—O—R), 1190—1220 (—$SO_3^{\ominus}$, $\phi$—O—R), 1670 $cm^{-1}$ (—CHO).

(ii) Synthesis of bis(4-N,N-diethylaminophenyl)-3,4-disodium sulfopropoxyphenyl methane (BSdiproPM)

7.0 g (16.4 mmol) of the 3,4-disodium sulfopropoxybenzaldehyde obtained in (i), 7.3 g (49.2 mmol) of N,N-diethylaniline and 4.5 g of zinc chloride were suspended in 140 ml of methyl cellosolve, the reaction was carried out at an internal temperature of 125°C for 28 hours. During the reaction, produced water was distilled off. After the termination of the reaction, 300 ml of dimethyl sulfoxide were added to dissolve the reaction product, and insoluble matters were filtered off. 1,700 ml of ethyl acetate were added to the filtrate to precipitate crystals. The crystals were filtered out and were dissolved in 15 ml of water. This solution was subjected to purification by a column chromatography (carrier Wakogel® C—200), and the eluent was subjected to distillation. The residue was dissolved in water, which was decolorized and filtered. After concentration of the filtrate, acetone was added to the concentrate to precipitate crystals, which were filtered out to obtain 2.68 g of the intended fine slightly blue crystals (yield: 23.2%).

(Elementary Analysis)

| | H | C | N |
|---|---|---|---|
| Calculated (%) | 6.27 | 56.08 | 3.96 |
| Found (%) | 6.46 | 56.06 | 4.14 |

UV (0.1 M tris buffer solution, pH = 7.5): $\lambda_{max}$ ($\varepsilon$) = 620 nm (166,300), IR (KBr): $\nu$ = 1020—1030 ($-SO_3^{\ominus}$, $\phi-O-R$), 118—1200 ($-SO_3^{\ominus}$, $\phi-O-R$), 1380 [$-N-(C_2H_5)_2$], 2950 $cm^{-1}$ ($-C_2H_5$).

Example 2

Synthesis of BSproPM

In accordance with Example 1 (ii), 2.5 g of sodium sulfopropoxybenzaldehyde obtained from p-hydroxybenzaldehyde and propanesultone similarly to Example 1 (i) were reacted with 3.4 g of N,N-diethylaniline in 50 ml of methyl cellosolve in the presence of 2.4 g of zinc chloride, and post-treatment was carried out similarly to Example 1 (ii) to obtain 0.7 g of light blue crystals of bis(4-N,N-diethylaminophenyl)-4-sodium sulfopropoxyphenyl methane (BSproPM) (yield: 13.6%).

(Elementary Analysis)

| | H | C | N |
|---|---|---|---|
| Calculated (%) | 7.19 | 65.91 | 5.12 |
| Found (%) | 7.33 | 65.94 | 5.28 |

UV (0.1 M tris buffer solution, pH = 7.5): $\lambda_{max}$ ($\varepsilon$) = 620 nm (121,800).

Example 3

Measurement of serum monoamine oxidase activity

Using 15 mM of allylamine as a substrate, uricase, BSdiproPM, Emal® NC (Kao Atlas Co., Ltd.; trademark) and POD were dissolved in 20 mM of phosphate buffer solution (pH = 7.0) to give the respective concentrations of 200 U/l, 0.03 mM, 5% and 3,000 U/l to prepare a substrate color test liquid.

A 8.9 mM aqueous solution of sodium diethyldithiocarbamate was prepared as a reaction termination liquid.

3 ml of the above substrate color test liquid were added to 50 µl of a sampled serum, and incubated at 37°C for 30 minutes. Then, 50 µl of the reaction terminator liquid were mixed thereinto and absorbance at a wavelength of 620 nm measured by using a reagent blank as control.

Using bovine monoamine oxidase manufactured by Sigma Co., Ltd., standard liquids of 5 IU/l, 10 IU/l and 20 IU/l were prepared, and then absorbances were measured with respect to these standard liquids as in the case of the serum to obtain a calibration curve therefrom, which is shown in Fig. 1.

The activity of monoamine oxidase in the sample was determined from the calibration curve.

Reference Example 1

Measurement of $H_2O_2$ by using a conventional coloring reaction

(Buffer solution)

30 mM of allylamine, 0.53 mM of phenol, and a surface active agent were added to 25 mM of Good buffer solution (pH 6.75) to prepare a buffer solution.

(First test liquid)

170 units of lipoprotein lipase, 425 units of ascorbate oxidase, 255 units of peroxidase and a stabilizer were added to 85 ml of the above prepared buffer solution to prepare a first test liquid.

(Second test liquid)

7.3 µmol of 10-N-methylcarbamoyl-3,7-dimethylamino-10H-phenothiazine (MCDP) and a stabilizer

were added to 85 ml of the above prepared buffer solution to prepare a second test liquid. For practical use, the first test liquid and the second test liquid were mixed with each other in the same volume to prepare a color test liquid.

An aqueous solution of 8.9 mM of sodium diethyldithiocarbamate was prepared as a reaction termination liquid.

3.0 ml of the color test liquid were preliminarily incubated in the constant temperature chamber at 37°C for about 5 min., and 50 µl of serum were added thereto, incubated at 37°C for 30 min. Then, 50 µl of the reaction terminator liquid were added to and mixed with the reaction solution, and absorbance (Es) at a wavelength of 660 nm was measured with reference to water as control.

By using 50 µl of monoamine oxidase standard liquids (prepared in Example 3) and 50 µl of purified water instead of the serum, absorbances Estd and $E_B$ were obtained as in the same procedure as above, and the activity of the monoamine oxidase was calculated by the following formula:

$$\text{Monoamine oxidase activity (IU/l)} = \frac{Es - E_B}{Estd - E_B} \times \text{standard enzyme unit}$$

Table 1 shows the changes in the reagent blank during the storage of the color test liquid (stored at 15°C) in the cases of Example 3 and Reference Example 1.

TABLE 1

| Stored time (hour) | Example 3 | Reference Example 1 |
|---|---|---|
| 0 | 0.030 | 0.027 |
| 2 | 0.030 | 0.041 |
| 5 | 0.029 | 0.124 |
| 7 | 0.030 | 0.172 |
| 24 | 0.031 | could not be measured |

As shown in Table 1, in the case of Reference Example 1, since the reagent is increasingly colored during the storage of the color test liquid and the reagent blank change increases with the lapse of time, it is necessary to prepare a fresh color test liquid for actual use. In contrast thereto, the color test liquid according to the present invention does not undergo a color change even after 24 hours.

Table 2 shows a comparison in measurement results between Example 3 and Reference Example 1.

TABLE 2

| Serum No. | Example 3 (IU/l) | Reference Example 1 (IU/l) |
|---|---|---|
| 1 | 1.5 | 1.7 |
| 2 | 7.8 | 7.2 |
| 3 | 2.3 | 2.3 |
| 4 | 1.1 | 1.4 |
| 5 | 4.2 | 3.9 |
| 6 | 1.6 | 1.6 |
| 7 | 5.1 | 4.8 |
| 8 | 12.7 | 13.0 |
| 9 | 0.9 | 1.1 |
| 10 | 1.2 | 1.0 |
| average | 3.84 | 3.80 |

As shown in Table 2, the values in Example 3 are well correlated with Reference Example 1, and no significant differences therebetween are observed (γ = 0.997).

Example 4

Quantitative analysis of free cholesterol in serum

BSdiproPM, uricase, cholesterol oxidase, peroxidase, Triton-X—100, and Emal® NC were dissolved in 0.05 M of phosphate buffer solution (pH 7.0) to give concentrations of 0.05 mM, 300 U/l, 100 U/l, 0.15% and 0.05% respectively and to prepare a color test liquid.

3 ml of the above color test liquid were added to 10 µl, of sampled serum, which was incubated at 37°C for 10 min. Then, absorbance at a wavelength of 620 nm was measured with reference to a reagent blank as control.

Separately, cholesterol standard liquids were prepared having concentrations of 25, 50, 100, 150 and 200 mg/dl respectively, and absorbances were measured in the same manner as in the serum to obtain a calibration curve therefrom. Fig. 2 shows a calibration curve.

The concentration of the cholesterol in the serum was determined from the calibration curve.

Reference Example 2

Quantitative analysis of free cholesterol in serum using known coloring reagent

4-Aminoantipyrine, phenol, cholesterol oxidase, peroxidase and Triton-X—100 were dissolved in 0.05 M phosphate buffer liquid (pH = 7.0) to give concentrations of 0.01%, 0.1%, 100 U/l, 3,000 U/l and 0.1% respectively and to prepare a color test liquid.

3 ml of the above color liquid were added to 50 µl of sampled serum, which was incubated at 37°C for 10 min. Then, absorbance at a wavelength of 505 nm was measured with reference to a reagent blank as control.

Separately, color was developed by using the cholesterol standard solutions (prepared in Example 4) in the same manner as above, and absorbances were measured to obtain a calibration curve therefrom. Fig. 2 shows the thus obtained calibration curve.

Table 3 shows a comparison in measurement results between Example 4 and Reference Example 2.

TABLE 3

| Serum No. | Measured value of cholesterol<br>Example 4<br>mg/dl | <br>Reference Example 2<br>mg/dl | No *1<br>uricase | Uric *2<br>acid<br>concentration |
|---|---|---|---|---|
| 1 | 41.8 | 43.1 | 20.7 | 4.1 |
| 2 | 34.0 | 34.7 | 5.4 | 8.5 |
| 3 | 40.8 | 43.1 | 17.0 | 4.8 |
| 4 | 29.3 | 30.6 | 6.2 | 5.7 |
| 5 | 62.3 | 62.1 | 35.2 | 6.3 |
| 6 | 64.6 | 63.3 | 40.6 | 4.3 |
| 7 | 27.8 | 28.6 | 1.7 | 9.3 |
| 8 | 54.1 | 50.4 | 22.9 | 5.2 |
| Average | 44.34 | 44.49 | | |

Note: *1 . . . "No uricase" means a case where measurement was carried out acording to Example 4 with respect to a color test liquid prepared from the components of the color test liquid of Example 4 with uricase being excluded therefrom.

*2 . . . "Uric acid concentration" means the concentration of the uric acid measured by using Uric Acid B-Test wako (Manufactured by Wako Pure Chemical Industries, Ltd.)

The significant difference in the measurement values between Example 4 and Reference Example 2 was examined using the t-test. The significant level was 5%, and no difference therebetween was observed. In the case of "no uricase", the measurement values were largely lowered due to the presence of uric acid in the serum, and the negative influence due to the uric acid is obvious.

Example 5

Quantitative analysis of hydrogen peroxide

BSdiproPM, peroxidase and Triton X—100 were dissolved in 0.05 M phosphate buffer solution (pH = 7.0) to give concentrations of 0.05 mM, 3,000 U/l and 0.05% respectively and to prepare a color test liquid.

3 ml of the above color test liquid were added to 20 μl of a sample containing 1—60 ppm of $H_2O_2$, which was incubated at 37°C for 10 min. Then, absorbance at a wavelength of 620 nm was measured with reference to a reagent blank as control.

Separately, by using hydrogen peroxide standard solutions prepared to be at concentrations of 15, 30, 45, and 60 ppm respectively, absorbances thereof were measured in the same manner as above to obtain a calibration curve therefrom. Fig. 3 shows the thus obtained calibration curve.

The concentration of the hydrogen peroxide in the sample was determined from the calibration curve of hydrogen peroxide.

Example 6

Quantitative analysis of hydrogen peroxide

BSproPM, peroxidase, and Triton X—100 were dissolved in 0.05 M of photophosphate buffer solution (pH = 7.0) to give concentrations of 0.05 mM, 3,000 U/l and 0.05% respectively and to prepare a color test liquid.

Absorbance of the sample was measured in the same manner as in Example 5, and the concentration of the hydrogen peroxide in the sample was determined from a calibration curve obtained by using the separately prepared hydrogen peroxide standard solution (used in Example 5).

Fig. 4 shows the calibration curve thus obtained.

Industrial Applicability

As mentioned above, since the novel coloring reagent according to the present invention is high in sensitivity, and the invention can effectively avoid the influences of the substances interfering with the color development, the color reagent of the invention is suitable for the quantitative measurement of a small amount of the component in a sample from a living organism, particularly a body fluid such as blood or urine. Further, since the coloring sensitivity can be appropriately adjusted according to the present invention, the use range at which the novel coloring reagent according to the present invention may be used is not limited to the quantitative measurement of the small amount of the component. Thus, they can be applied to a wide range of uses such as clinically chemical analysis by utilizing the excellent characteristics that the coloring wavelength is on a long wavelength side and no color fading occurs with lapse of time after the color is developed.

**Claims**

1. A triphenyl methane derivative represented by the general formula (I):

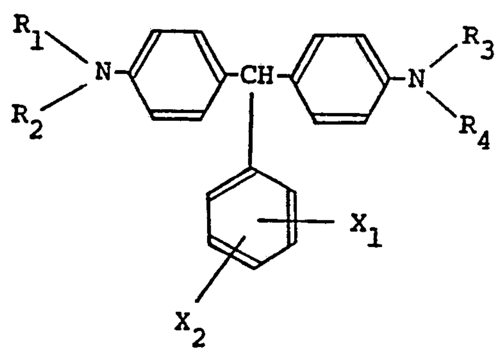

(I)

wherein $R_1$, $R_2$, $R_3$ and $R_4$, which may be the same as or different from one another, represent $C_{1-3}$-alkyl groups, and $X_1$ and $X_2$ both represent $—O(CH_2)_nSO_3M$ in which M is a hydrogen atom, an alkali metal ion or $NH_4^+$, and n is an integer of 2—4, or either of $X_1$ and $X_2$ represents $—O(CH_2)_nSO_3M$ in which M and n are the same meanings as given above and the other represents a hydrogen atom.

2. The compound according to Claim 1, wherein both of $X_1$ and $X_2$ represent $—O(CH_2)_nSO_3M$.

3. The compound according to Claim 1, wherein either of $X_1$ and $X_2$ represents $—O(CH_2)_nSO_3M$, and the other represent a hydrogen atom.

4. The compound according to Claim 1, wherein the derivative is bis(4-N,N-diethylaminophenyl)-4-sodiumsulfopropoxyphenyl methane or bis(4-N,N-diethylaminophenyl)-3,4-disodium sulfopropoxyphenyl methane.

5. A method of quantitatively measuring an oxidative substance by using as a coloring component a triphenyl methane derivative represented by the general formula (I):

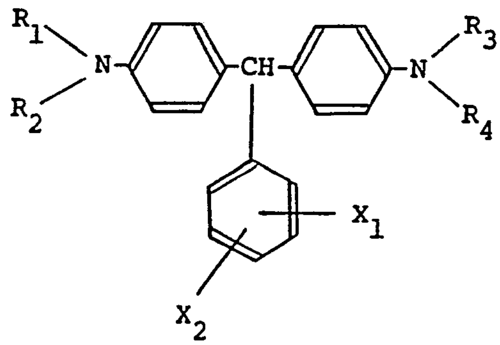

(I)

wherein $R_1$, $R_2$, $R_3$ and $R_4$, which may be the same as or different from one another, represent $C_{1-3}$-alkyl groups, and $X_1$ and $X_2$ both represent $—O(CH_2)_nSO_3M$ in which M is a hydrogen atom, an alkali metal ion or $NH_4^+$, and n is an integer of 2—4, or either of $X_1$ and $X_2$ represents $—O(CH_2)_nSO_3M$ in which M and n are the same meanings as given above and the other represents a hydrogen atom.

6. The method of quantitatively measuring an oxidative substance according to Claim 5, wherein the coloring component is oxidized and colored in the presence of a peroxidase, and the color thus developed is colorimetrically determined.

7. The method of quantitatively measuring an oxidative substance according to Claim 6, wherein the oxidative substance is hydrogen peroxide, and said hydrogen peroxide is hydrogen peroxide which is generated through an enzyme reaction.

8. The method of quantitatively measuring an oxidative substance according to Claim 7, wherein the hydrogen peroxide is hydrogen peroxide which is generated through the enzyme reaction in the quantitative measurement of an extremely small amount of a component in a sample from a living organism.

9. The method of quantitatively measuring an oxidative substance according to Claim 8, wherein the quantitative measurement of the extremely small amount of the component in the sample from the living organism is a quantitative measurement of a substrate or enzyme activity in the sample from the living organism, that an oxidation enzyme is acted upon the substrate or a substance produced through the enzyme reaction to generate hydrogen peroxide, and the thus generated hydrogen peroxide is quantitatively measured.

**Patentansprüche**

1. Ein Triphenylmethanderivat, dargestellt durch die allgemeine Formel (I):

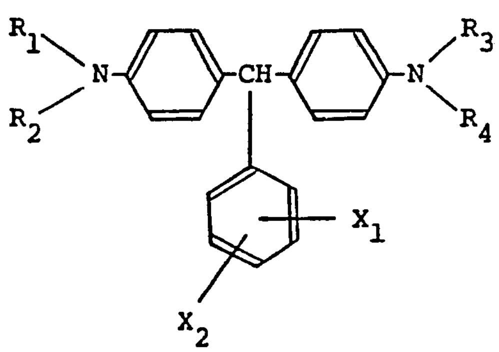

(I)

in welcher $R_1$, $R_2$, $R_3$ und $R_4$, welche gleich oder verschieden sein können, $C_{1-3}$-Alkylgruppen darstellen, und $X_1$ und $X_2$ jeweils $—O(CH_2)_nSO_3M$ bedeuten, wobei M ein Wasserstoffatom, ein Alkalimetallion oder $NH_4^+$ bedeutet, und n eine ganze Zahl von 2 bis 4 ist, oder eine der Gruppen $X_1$ und $X_2$ $—O(CH_2)_nSO_3M$ bedeuten, wobei M und n die oben genannten Bedeutungen haben, und die andere Gruppe ein Wasserstoffatom ist.

2. Die Verbindung nach Anspruch 1, in welcher beide Gruppen $X_2$ und $X_2$ $—O(CH_2)_nSO_3M$ darstellen.

3. Die Verbindung nach Anspruch 1, in welcher eine der beiden Gruppe $X_1$ und $X_2$ $—O(CH_2)_nSO_3M$ ist, und die andere Gruppe eine Wasserstoffatom darstellt.

4. Die Verbindung nach Anspruch 1, in welcher das Derivat Bis(4-N,N-diäthylaminophenyl)-4-

natriumsulfopropoxyphenylmethan oder Bis(4-N,N-diäthylaminophenyl)-3,4-dinatriumsulfopropoxyphenylmethan ist.

5. Eine Verfahren zum quantitativen Messen einer oxidativen Substanz unter Verwendung eines Triphenylmethanderivats als Färbungskomponente, dargestellt durch die allgemeine Formel (I):

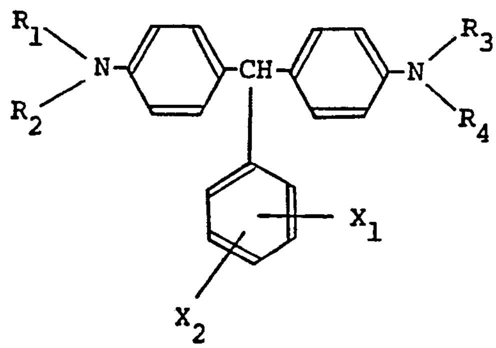

(I)

in welcher $R_1$, $R_2$, $R_3$ und $R_4$, welche gleich oder verschieden sein können, $C_{1-3}$-Alkylgruppen darstellen, und $X_1$ und $X_2$ jeweils $—O(CH_2)_nSO_3M$, wobei M ein Wasserstoffatom, ein Alkalimetallion oder $NH_4^+$ ist, bedeuten und n eine ganze Zahl von 2 bis 4 ist, oder eine der beiden Gruppen $X_1$ und $X_2$ $—O(CH_2)_nSO_3M$ ist, wobei M und n die vorstehend genannten Bedeutungen haben, und die andere Gruppe ein Wasserstoffatom darstellt.

6. Das Verfahren des quantitativen Messens einer oxidative Substanz nach Anspruch 5, in welchem die Färbungskomponente in Gegenwart von Peroxidase oxidiert und gefärbt wird, und die so entstehendene Farbe colorimetrisch bestimmt wird.

7. Das Verfahren des quantitativen Messens einer oxidative Substanz nach Anspruch 6, in welchem die oxidative Substanz Wasserstoffperoxid ist, und das genannte Wasserstoffperoxid ein durch eine Enzymreaktion erzeugtes Wasserstoffperoxid ist.

8. Das Verfahren des quantitativen Messens einer oxidative Substanz nach Anspruch 7, in welchem das Wasserstoffperoxid eine Wasserstoffperoxid ist, das durch die Enzymreaktion beim quantitativen Messen einer äußerst kleinen Menge einer Komponente in einer Probe aus einem lebenden Organismus gebildet wurde.

9. Das Verfahren des quantitativen Messens einer oxidative Substanz nach Anspruch 8, in welchem das quantitative Messen der äußerst kleinen Menge der Komponente in der Probe aus dem lebenden Organismus ein quantitatives Messen einer Substrat- oder Enzymaktivität in der Probe aus dem lebenden Organismus ist, daß man ein Oxidationsenzym auf das Substrat oder eine durch die Enzymreaktion hergestellte Substanz einwirken läßt, um Wasserstoffperoxid zu erzeugen, und daß das so gebildete Wasserstoffperoxid quantitativ gemessen wird.

**Revendications**

1. Dérivé de triphénylméthane représenté par le formule générale (I):

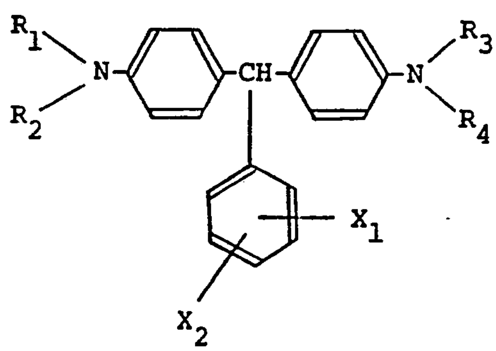

(I)

dans laquelle $R_1$, $R_2$, $R_3$ et $R_4$, qui peuvent être semblables ou différents l'un de l'autre représentent des groupes alkyles en $C_{1-3}$ et $X_1$ und $X_2$ représentent tous deux $—O(CH_2)_nSO_3M$, où M est un atome d'hydrogène, un ion de métal alcalin ou $NH_4^+$ et n est un entier valant de 2 à 4, ou l'un de $X_1$ et $X_2$ représente $—O(CH_2)_nSO_3M$, où M et n ont la même signification que ci-dessus, et l'autre représente un atome d'hydrogène.

2. Composé selon la revendication 1, dans lequel $X_1$ und $X_2$ représentent tous deux $—O(CH_2)_nSO_3M$.

3. Composé selon la revendication 1, dans lequel l'un de $X_1$ et $X_2$ représente $—O(CH_2)_nSO_3M$ et l'autre représente un atome d'hydrogène.

4. Composé selon la revendication 1, qui est le bis(4-N,N-diéthylaminophényl)-4-sodium sulfopropoxyphényle méthane ou le bis(4-N,N-diéthylaminophényl)-3,4-disodium sulfopropoxyphényle méthane.

5. Procédé de mesure quantitative d'une substance oxydante par utilisation, comme composant colorant, d'un dérivé de triphénylméthane représente par la formule générale (I):

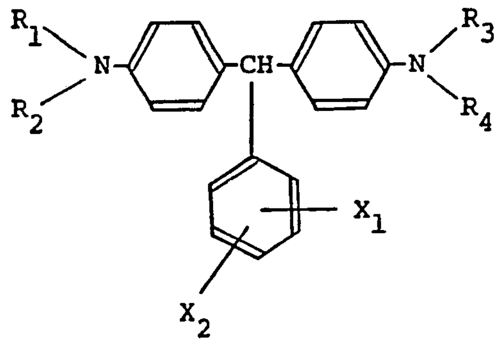

(I)

dans laquelle $R_1$, $R_2$, $R_3$ et $R_4$, qui peuvent être semblables ou différents l'un de l'autre, représentent des groupes alkyles en $C_{1-3}$ et $X_1$ und $X_2$ représentent tous deux $—O(CH_2)_nSO_3M$, où M est un atome d'hydrogène, un ion de métal alcalin ou $NH_4^+$ et n est un entier valant de 2 à 4, ou l'un de $X_1$ et $X_2$ représente $—O(CH_2)_nSO_3M$, où M et n ont la même signification que ci-dessus, et l'autre représente un atome d'hydrogène.

6. Procédé de mesure quantitative d'une substance oxydante selon la revendication 5, dans lequel le composant colorant est oxydé et coloré en présence d'une peroxydase, et la couleur ainsi formée est déterminée colorimétriquement.

7. Procédé de mesure quantitative d'une substance oxydante selon la revendication 6, dans lequel la substance oxydante est le peroxyde d'hydrogène, et ce peroxyde d'hydrogène est du peroxyde d'hydrogène formé par une réaction enzymatique.

8. Procédé de mesure quantitative d'une substance oxydante selon la revendication 7, dans lequel lequel le peroxyde d'hydrogène est du peroxyde d'hydrogène formé par la réaction enzymatique dans la mesure quantitative d'une quantité extrêmement faible d'un composant d'un échantillon provenant d'un organisme vivant.

9. Procédé de mesure quantitative d'une substance oxydante selon la revendication 8, dans lequel la mesure quantitative de la quantité extrêmement faible du composant dans l'échantillon provenant de l'organisme vivant est une mesure quantitative d'un substrat ou d'une activité enzymatique dans l'échantillon provenant de l'organisme vivant, on fait agir une enzyme oxydante sur le substrat ou une substance produite par la réaction enzymatique, pour produire un peroxyde d'hydrogène, et le peroxyde d'hydrogène ainsi formé est mesuré quantitativement.

FIG.1

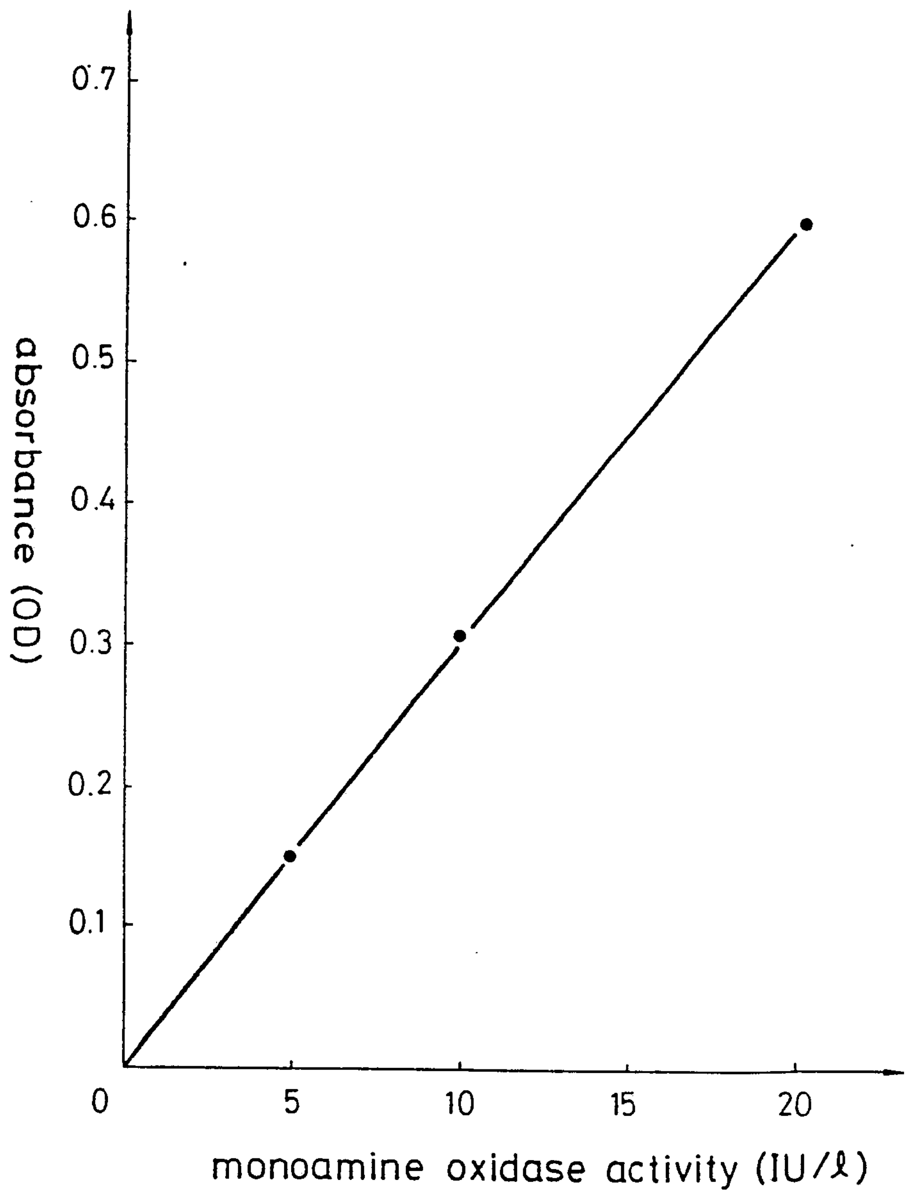
0.7
0.6
0.5
0.4
0.3
0.2
0.1
absorbance (OD)
0
5
10
15
20
monoamine oxidase activity (IU/ℓ)

FIG.2

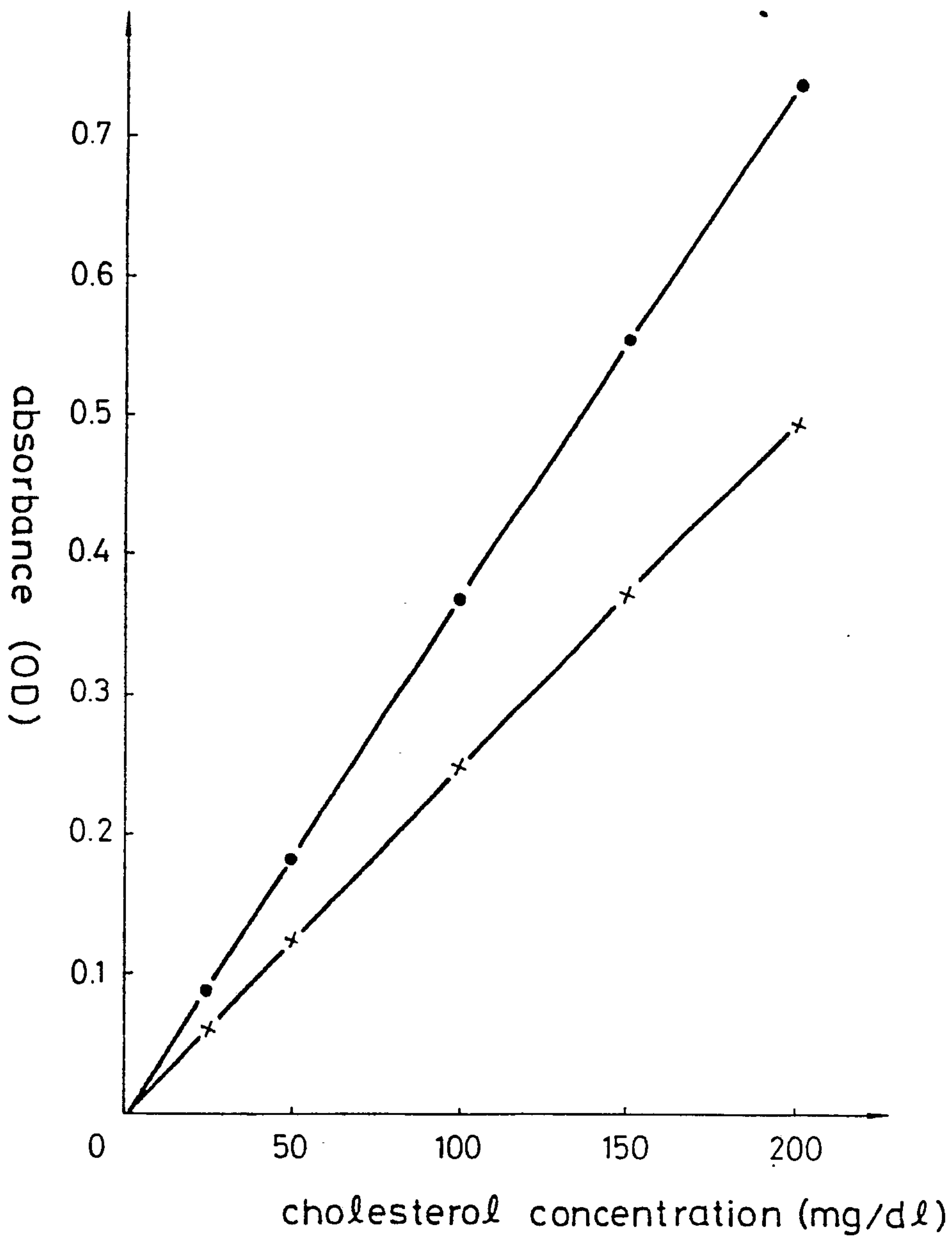
0.7
0.6
0.5
0.4
0.3
0.2
0.1
absorbance (OD)
0
50
100
150
200
cholesterol concentration (mg/dℓ)

FIG.3

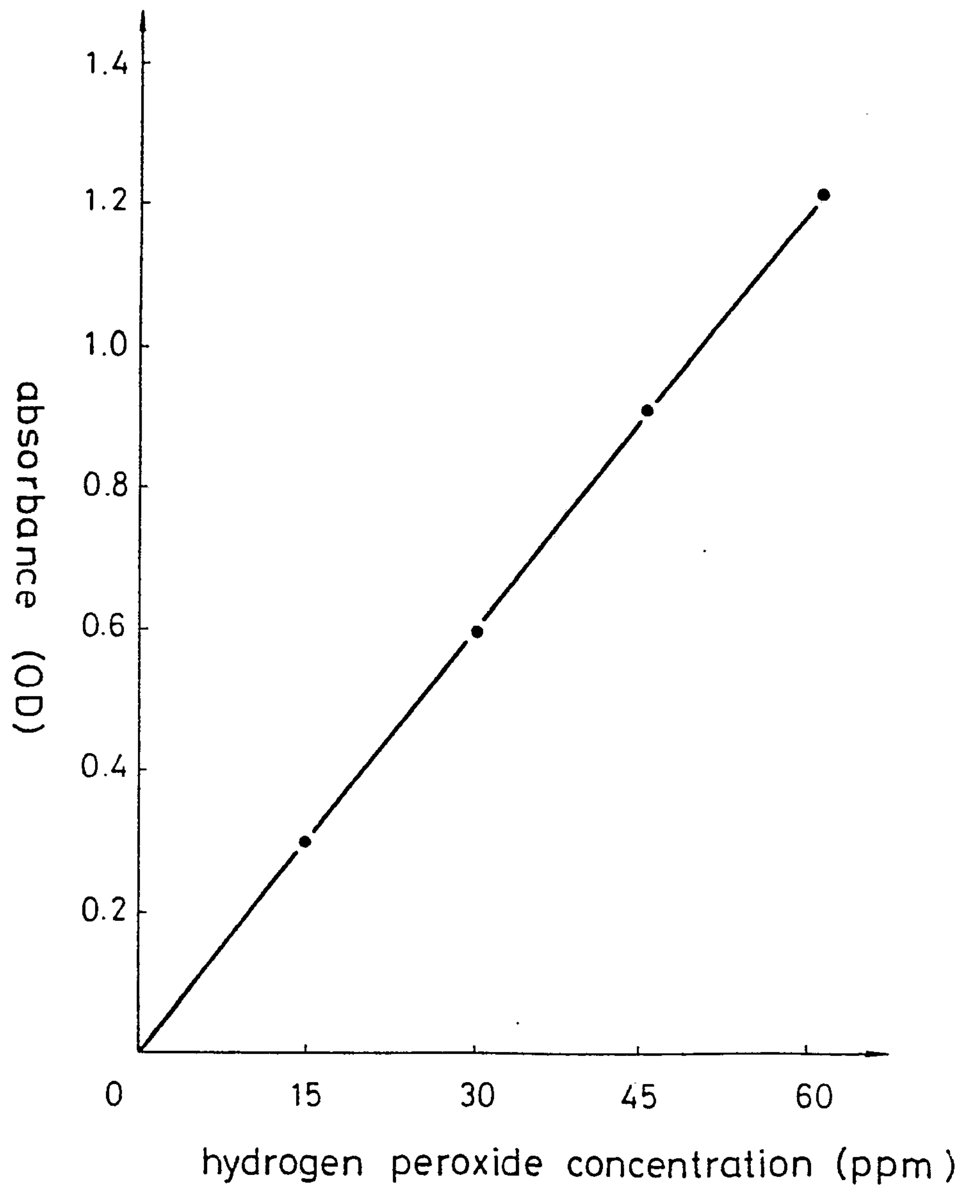
1.4
1.2
1.0
0.8
0.6
0.4
0.2
absorbance (OD)
0
15
30
45
60
hydrogen peroxide concentration (ppm)

FIG.4

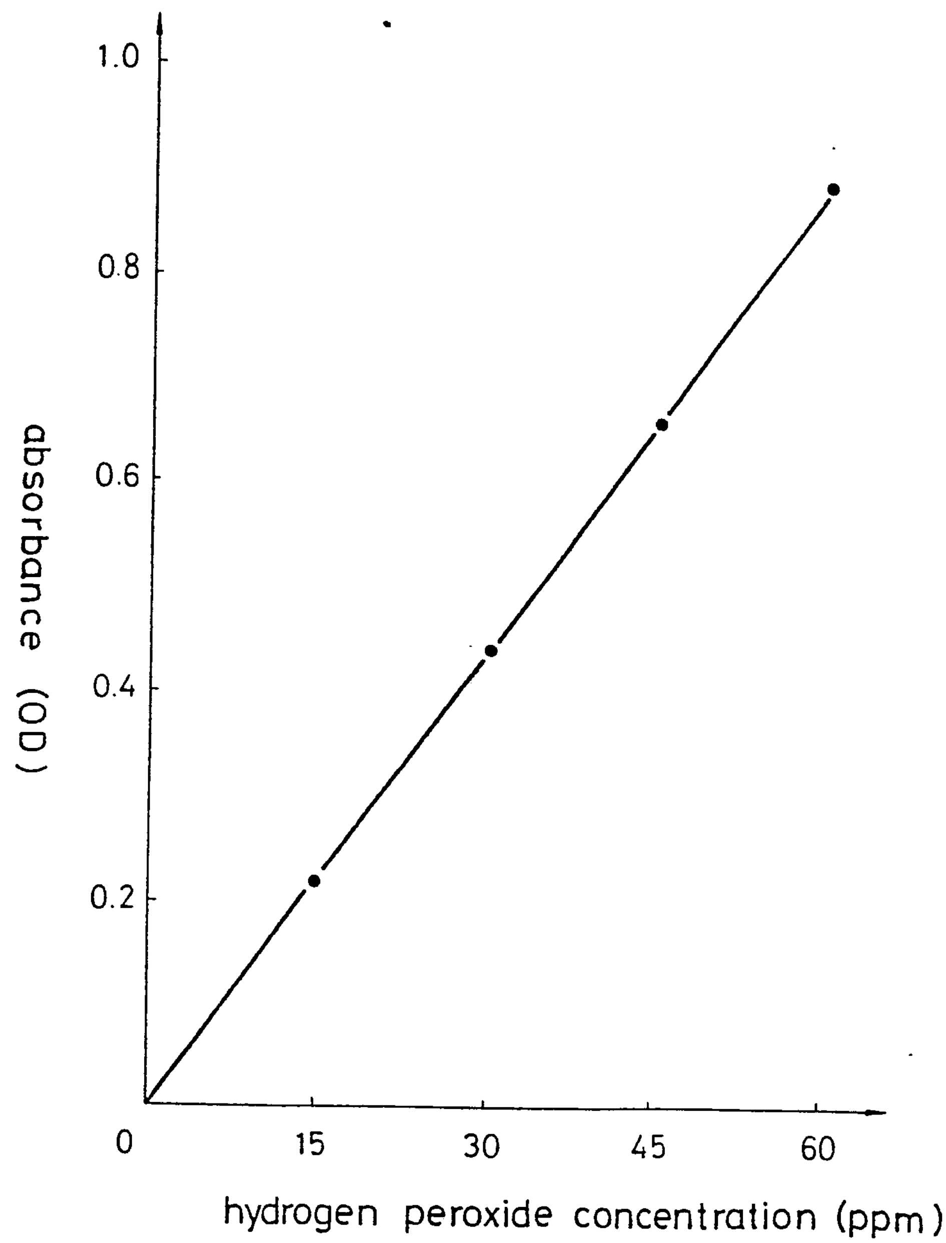
1.0
0.8
0.6
0.4
0.2
absorbance (OD)
0
15
30
45
60
hydrogen peroxide concentration (ppm)